Europäisches Patentamt

European Patent Office

Office européen des brevets

(19)

(11) Publication number: 0 244 178
A2

# (12) EUROPEAN PATENT APPLICATION

(21) Application number: 87303676.8

(22) Date of filing: 27.04.87

(51) Int. Cl.⁴: **A 61 K 47/00**
**A 61 K 9/08**

(30) Priority: 28.04.86 US 856390

(43) Date of publication of application:
04.11.87 Bulletin 87/45

(84) Designated Contracting States:
AT BE CH DE ES FR GB GR IT LI LU NL SE

(71) Applicant: IOLAB, INC
500 Iolab Drive
Claremont California 91711 (US)

(72) Inventor: Keller, Nancy
2144 Webster Street
Palo Alto CA 94301 (US)

Olejnik, Orest
4433 Borina Drive
San Jose CA 95129 (US)

Abelson, Mark Barry
26 Phillips Street
Andover MA 01810 (US)

(74) Representative: Jones, Alan John et al
CARPMAELS & RANSFORD 43 Bloomsbury Square
London, WC1A 2RA (GB)

(54) Intraocular dosage compositions and method of use.

(57) A controlled release injectable intraocular dosage composition comprises an aqueous solution of
a pharmaceutical compound or non-toxic pharmaceutically acceptable salt thereof and
an ophthalmologically acceptable, non-toxic, viscosity-increasing polysaccharide.
The polysaccharide is preferably a hyaluronic acid or a mixture of a hyaluronic acid and chondroitin sulfate.

**Description**

INTRAOCULAR DOSAGE COMPOSITIONS AND METHOD OF USE

BACKGROUND OF THE INVENTION

Field of the Invention:

This invention relates to intraocular administration of medication and more particularly to compositions for administering medication to the interior of the eye.

Description of the Prior Art:

The treatment of disease occurring in tissues within the eye presents a difficult problem for the physician. Many of the tissues are very delicate and easily injured by surgical intervention, and treatment with medication is also difficult because many of the ocular tissues are not heavily supplied with blood. Particularly serious problems are encountered in cases of bacterial endophthalmitis wherein it is difficult to supply antibiotics to the focus of the infection.

Accordingly, it is conventional to supply a medicament to treat an ophthalmic disease by systemic administration, in the hope that a sufficient amount of the medication will reach the target tissue in the eye through the general circulation, or by topical administration, e.g., in the form of eye drops or ointment, in the hope that enough medication will reach the target tissue by diffusion and/or local circulation.

Evidently each of these methods has drawbacks. Since the transfer of pharmaceutical compounds from the blood to many ocular tissues, e.g., the cornea, the vitreous, and the like, is inefficient, administration of ophthalmic drugs through the general circulation requires a blood concentration of the pharmaceutical compound which is relatively high and may induce undesired side effects elsewhere in the body. Similarly, transfer of a topically applied pharmaceutical from the surface of the eye to the interior tissues is not only inefficient, but the also tends to be washed away by the flow of tears. Consequently, topically applied medications do not exhibit optimum effectiveness.

Drugs have also been administered to the eye by periorbital injection, with the intention that the drug will reach the desired site in the eye by diffusion and local circulation. This method avoids loss of a portion of the pharmaceutical by the leaching action of the tear fluid, but suffers from the other drawbacks of topical administration.

Some attempts have been made to administer medication to the interior of the eye by direct injection of solutions of a pharmaceutical compound. While this procedure can place the medication in contact with the target tissue, if the medication is administered in the conventional injectable solution it may not remain in close contact with the tissue to be treated for a period of time sufficient to be effective, and it has the disadvantage that the eyeball must be punctured by a needle. Because of such trauma to the eye, physicians are reluctant to make injections within the eye, especially when several injections may be required to provide the needed duration of treatment.

Therefore, a need has continued to exist for a method of supplying medication to the interior of an eye by an efficient injection means without requiring multiple injections.

SUMMARY OF THE INVENTION

This goal has now been achieved by an injectable composition comprising an aqueous solution of a pharmaceutical compound or pharmaceutically acceptable non-toxic salt thereof containing an ophthalmologically acceptable, non-toxic, viscosity-increasing polysaccharide.

Accordingly, it is an object of the invention to provide an intraocular dosage form.

A further object is to provide an intraocular dosage form capable of sustained release of a pharmaceutical compound.

A further object is to provide an intraocular dosage form incorporating a polysaccharide.

Further objects of the invention will become apparent from the description which follows.

BRIEF DESCRIPTION OF THE FIGURES

Figure I shows the overall improvement in test scores relative to the saline controls over the seven days of the experiment of the Example.

Figure 2 shows the relative intraocular pressure (IOP) after 7 days relative to the IOP before challenge.

Figure 3 shows the protein concentration in the aqueous humor after 7 days.

Figure 4 shows the numbers of polymorphonuclear leukocytes (PMN) in the aqueous humor after 7 days.

DETAILED DESCRIPTION OF THE INVENTION AND PREFERRED EMBODIMENTS

The intraocular dosage composition of this invention is implanted within the globe of the eye in contact with or as close as possible to the tissue to be treated. The viscous dosage composition of the invention will not itself diffuse away from the injection site and its viscosity prevents diffusion of the pharmaceutical away from the injection site. Rather the composition of this invention allows a slow diffusion of the pharmaceutical from the injected dose to the target tissue, thereby assuring a controlled release of the medication so as to provide the target tissue with a therapeutically effective dose of the drug for a period of time longer than a conventional injection using a non-viscous vehicle for the drug.

The viscosity-increasing polysaccharide component of the composition of this invention may be any polysachharide which does not produce adverse reactions when introduced into the intraocular enviroment. In particular, the polysaccharide should not be an antigen which will provoke an immune response by the organism, with consequent damage to the surrounding tissue. A preferred viscosity-increasing polysaccharide is hyaluronic acid, which is a naturally-occurring polymer of D-glucuronic acid and N-acetyl-D-glucosamine. Hyaluronic acid is found in animal tissues such as umbilical cord, vitreous humor, synovial fluid, rooster comb and the like, and also in certain bacteria, such as group A and C hemolytic streptococci. It is generally obtained on a commercial scale by extraction from umbilical cord or rooster combs. The molecular weight of the naturally occurring polymer ranges from about 50,000 to about 8,000,000, depending on the source and method of isolation. One type of hyaluronic acid which may be utilized is an ultrapure, non-inflammatory hyaluronic acid prepared as disclosed in Balasz, U.S. Patent 4,141,973. A particularly preferred hyaluronic acid is an ultrapure polymer having a molecular weight less than 750,000 which is dissolved in an isotonic aqueous solution substantially devoid of electrolytes. Such a solution has a sufficiently high viscosity to achieve the beneficial effects of the composition of this invention, and indeed has a viscosity comparable to that of a solution of a hyaluronic acid having a substantially higher molecular weight, but which is formulated with the conventional sodium chloride and phosphate buffer electrolytes.

The intraocular dosage composition of this invention should have a viscosity sufficient to prevent substantial diffusion of the drug contained therein from the site at which the composition is implanted. Evidently, the exact viscosity is not critical and the skilled practitioner can select the desired viscosity by incorporating more or less of the polysaccharide into the intraocular medication composition.

The pharmaceutical compound which is contained in the intraocular medication composition of the invention may be any drug which is used in treating a condition of the ocular tissues. For treating bacterial endophthalmitis the composition will contain an amount of an antibiotic effective to combat the bacterial infection. The amount will evidently vary according to the particular antibiotic used and the selection of the antibiotic and the amount thereof in the composition of the invention is within the routine capability of the skilled practitioner. Suitable antibiotics include penicillins, cephalosporins, aminoglycosides, e.g. gentamicin, tetracycline and the like. Antibiotics usable in the compositions and method of this invention may be found in the discussion of antibiotics found in Remington's Practice of Pharmacy, A. Osol, Ed., 16th Ed., Mack Publishing Co., Easton, Pennsylvania (1980), pages 1123-1146.

Another condition which is usefully treated by intraocular injection of the composition of this invention is intraocular inflammation, e. g., uveitis. For treatment of inflammatory conditions the composition of the invention will contain an antiinflammatory drug, e.g., a steroid or non-steroid antiinflammatory drug. The amount of drug used in the composition of the invention for treating inflammation will vary depending on the choice of drug and the extent and location of the inflammation within the eye. The selection of the antiinflammatory drug and the concentration to be used in the composition of the invention is within the routine competence of the skilled practitioner. Suitable antiinflammatory steroids may be found among those listed in Remington's Practice of Pharmacy, A. Osol, Ed., 16th Ed., Mack Publishing Co., Easton, Pennsylvania (1980), pages 901-912. Non-steroidal antiinflammatory drugs may be found in this work on pages 912-913. Typical steroidal antiinflammatory drugs include dexamethasone, beclomethasone, betamethasone and the like. Typical non-steroidal antiinflammatory drugs include indomethacin, ibuprofen, and the like

The invention will now be illustrated by the following example which compares the efficacy of intraocular medication by injection using a conventional isotonic saline vehicle and using the compositons of this invention. The example is only illustrative and is not intended to be interpreted as limiting the scope of the invention which is defined only by the appended claims.

Example

This example illustrates the antiinflammatory effect of dexamethasone when administered in the composition of this invention compared with its administration in a conventional saline solution.

An inflammatory condition of the eye was produced in the New Zealand White rabbit by the following procedure. Rabbits weighing 2.2 kg to 3.6 kg were used. They were sensitized to bovine serum albumin proteins by subcutaneous injections given three times a day at two day intervals. Three days after the last sensitizing dose one eye of each animal was challenged by an intravitreal injection of antigen. Prior to injection the eyes were anesthetized, the posterior portion of the globe was exposed and 0.05 ml of antigen solution was injected into the central part of the vitreous from an 0.1 ml Hamilton syringe via a 30 gauge nedle. Care was taken to avoid puncturing the exterior ocular musculature and visible blood vessels.

The result of the immunoreaction of the sensitized rabbits to the intravitreal challenge with antigen is an inflammation of the iris which can be observed and evaluated by conventional examination of the eye using a slit lamp. Twenty-four hours after challenge the eyes were examined by slit lamp and given a numerical score based on the observed congestion of the iris vessels, and the presence of iris edema. Before treatment was started the animals were sorted into groups by iris score, so that for each experiment the initial mean iris scores in the group for that experiment were similar. After the initial scoring and sorting of the animals into groups, the treatment was begun.

The inflammation was treated by administration of the steroid antiinflammatory drug dexamethasone. One group was treated by topical administration of dexamethasone in a commercially available suspension having a concentration of 0.1 %.

The inflammation was treated by intravitreal injection of physiological saline solution (control), dexamethasone sodium phosphate in saline, or dexamethasone sodium phosphate in an aqueous solution containing an amount of a hyaluronic acid to increase the viscosity of the injected solution by a substantial amount. The injections were made slowly from a 0.l ml Hamilton syringe through a 30 gauge needle through the posterior globe into the central part of the vitreous. Several doage levels were given namely 50 micrograms, l25 micrograms, 250 micrograms and 500 micrograms for the dexamethasone in saline, and 50 micrograms, l00 micrograms, 200 micrograms or 500 micrograms for the dexamethasone in polymer. Iris scores and intraocular pressure were determined post challenge at 28, 48, 72 and 96 hours and at 7 days. Aqueous humor protein concentration and polymorphonuclear leukocyte cell (PMN) counts were measured at 7 days post-challenge.

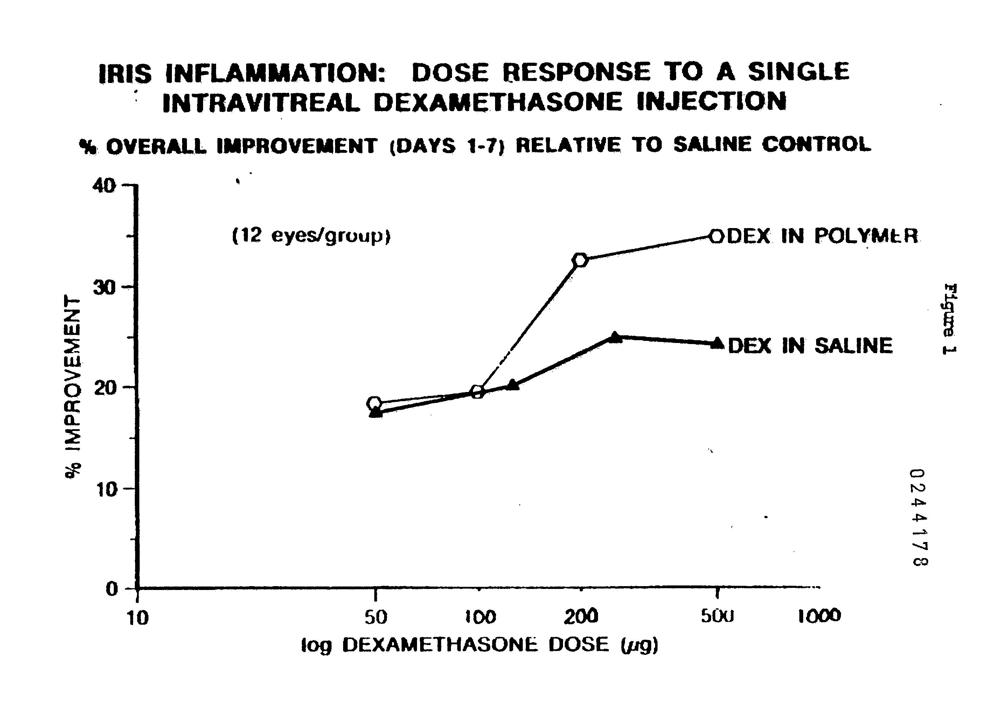

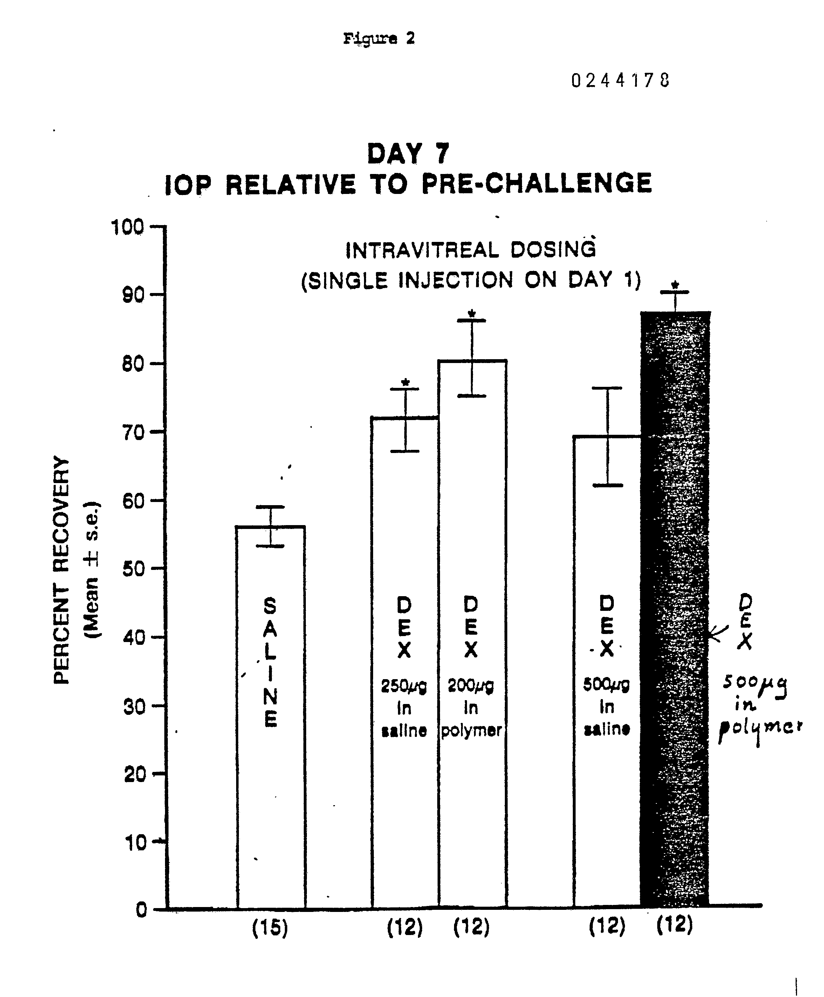

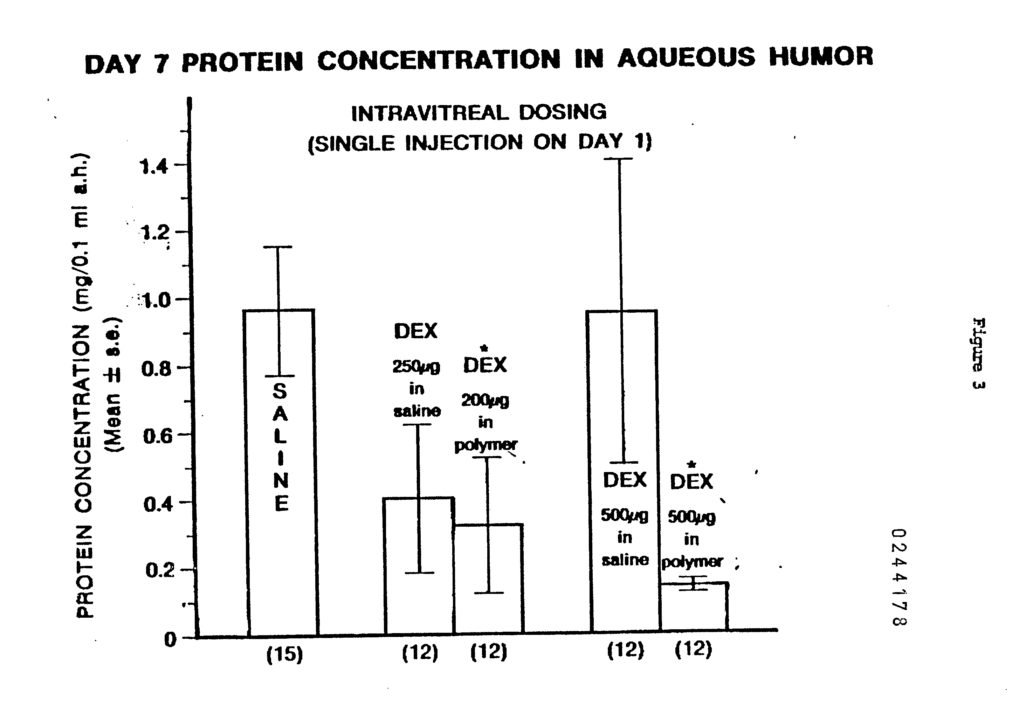

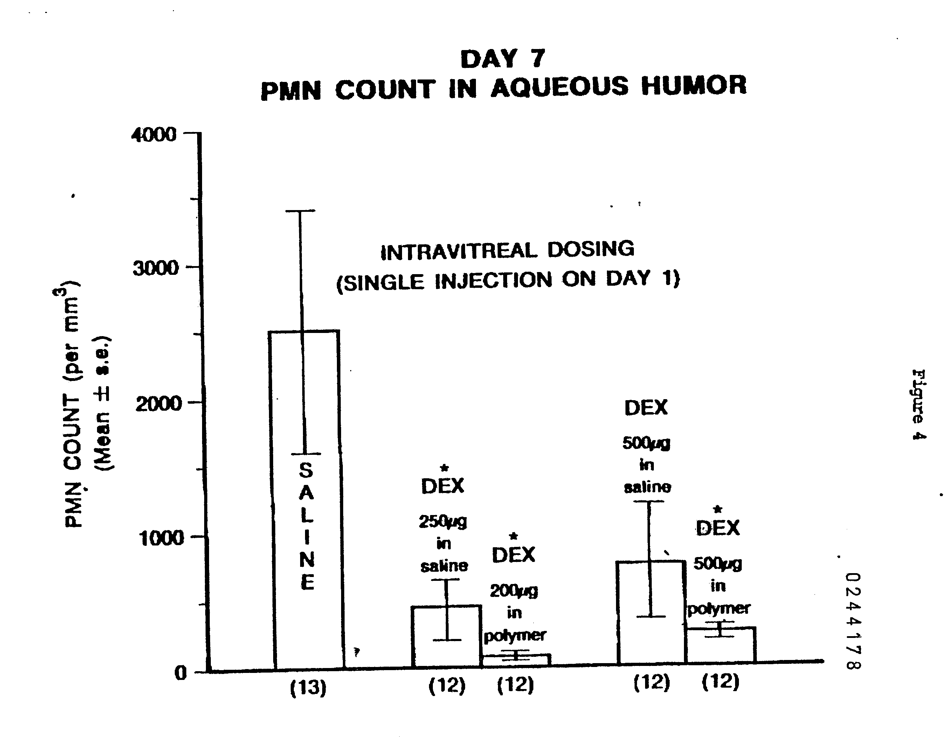

The results of the experiments are illustratedin Figures l - 4. Figure l shows that the dexamethasone administered in the viscous polymer solution produced a substantially increased improvement in the condition of the inflamed eye as compared with the improvement produced by the dexamethasone administered in saline solution. Figure 2 shows that the restoration of intraocular pressure (IOP) toward normal values is better when the dexamethasone is administered in the viscous polymer solution, especially at the higher dosage levels. Figure 3 shows that the amount of protein in the aqueous humor is substantialkly lower when the dexamethasone is administered in the polymer solution. Finally Figure 4 shows that the number of PMN's in the aqueous humor is lower when the dexamethasone is administered in the viscous polymer-containing dosage composition of this invention.

The invention having been fully described, it should be understood that it may be embodied in other specific forms or variations without departing from its spirit or essential characteristics. Accordingly, the embodiments described above are to be considered in all respects as illustrative and not restrictive; the scope of the invention being indicated by the appended claims rather than by the foregoing description, and all changes which come within the meaning and range of equivalency of the claims are intended to be embraced therein.

## Claims

l. A controlled release injectable intraocular dosage composition comprising an aqueous solution of

a pharmaceutical compound or non-toxic pharmaceutically acceptable salt thereof and

an ophthalmologically acceptable, non-toxic, viscosity-increasing polysaccharide.

2. The composition of claim l wherein said polysaccharide is a hyaluronic acid.

3. The composition of claim l or claim 2 wherein said polysaccharide is a hyaluronic acid having a molecular weight less than 750,000.

4. The composition of claim l wherein said polysaccharide is a mixture of a hyaluronic acid and a chondroitin sulfate.

5. The composition of any one of claims l to 4 wherein said pharmaceutical compound is an anti-inflammatory.

6. The composition of claim 5 wherein said pharmaceutical compound is a steroid.

7. The composition of claim 5 wherein said pharmaceutical compound is dexamethasone sodium phosphate.

8. The composition of any one of claims l to 4 wherein said pharmaceutical compound is an antibiotic.

9. The composition of any one of claims l to 8 for use in administering medication to the interior of the eye by intravitreous injecting.

**IRIS INFLAMMATION: DOSE RESPONSE TO A SINGLE INTRAVITREAL DEXAMETHASONE INJECTION**

% OVERALL IMPROVEMENT (DAYS 1-7) RELATIVE TO SALINE CONTROL

Figure 1

Figure 2

0 2 4 4 1 7 8

# DAY 7
# IOP RELATIVE TO PRE-CHALLENGE

INTRAVITREAL DOSING
(SINGLE INJECTION ON DAY 1)

**DAY 7 PROTEIN CONCENTRATION IN AQUEOUS HUMOR**

Figure 3

0244178

# DAY 7
# PMN COUNT IN AQUEOUS HUMOR

Figure 4